(19)

Europäisches Patentamt
European Patent Office
Office européen des brevets

(11)  **EP 4 114 250 B1**

(12)  **EUROPEAN PATENT SPECIFICATION**

(45) Date of publication and mention
of the grant of the patent:
**10.09.2025  Bulletin 2025/37**

(21) Application number: **21715675.1**

(22) Date of filing: **02.03.2021**

(51) International Patent Classification (IPC):
*A61B 5/00* *(2006.01)*    *A61B 5/022* *(2006.01)*
*A61B 5/024* *(2006.01)*    *A61B 5/026* *(2006.01)*

(52) Cooperative Patent Classification (CPC):
**A61B 5/022; A61B 5/0053; A61B 5/02416;**
**A61B 5/0261;** A61B 2562/0252

(86) International application number:
**PCT/IB2021/051740**

(87) International publication number:
**WO 2021/176358 (10.09.2021 Gazette 2021/36)**

(54) **METHOD FOR CUFF-LESS BEAT-TO-BEAT BLOOD PRESSURE ESTIMATION USING TWO RELATIVE BLOOD VOLUME SENSORS ON DIFFERENT APPLIED PRESSURES**

VERFAHREN ZUR MANSCHETTENLOSEN SCHLAG-ZU-SCHLAG-BLUTDRUCKSCHÄTZUNG UNTER VERWENDUNG VON ZWEI RELATIVEN BLUTVOLUMENSENSOREN AUF VERSCHIEDENEN ANGEWANDTEN DRÜCKEN

MÉTHODE D'ESTIMATION DE TENSION ARTÉRIELLE  DE BATTEMENT À BATTEMENT, SANS BRASSARD, À L'AIDE DE DEUX CAPTEURS DE VOLUME SANGUIN RELATIF SUR DIFFÉRENTES PRESSIONS APPLIQUÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **02.03.2020   US 202062983789 P**

(43) Date of publication of application:
**11.01.2023   Bulletin 2023/02**

(73) Proprietor: **Tallinn University of Technology
19086 Tallinn (EE)**

(72) Inventors:
• **PILT, Kristjan**
**19086 Tallinn (EE)**
• **KARAI, Deniss**
**19086 Tallinn (EE)**

(74) Representative: **Koppel, Mart Enn**
**KOPPEL Patendibüroo OÜ**
**Kajaka 4-10**
**13418 Tallinn (EE)**

(56) References cited:
**EP-A1- 3 342 336      WO-A1-2017/152098**
**US-A- 4 846 189      US-A1- 2017 209 053**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Technical field

The present invention relates to a novel method and a device for the non-occlusive continuous non-invasive determination of blood pressure using two blood volume sensors, which are under two different applied pressures. More specifically, the present invention relates to use of a non-linear function, which is newly updated for every cardiac cycle, to model the relationship between blood pressure and relative blood volume change.

### Background of the invention

10 The method proposed by J. Penaz's as so-called "volume-clamp" method as a possibility for continuous recording of blood pressure has been further developed by several authors. The common disadvantage of all devices operating on the "volume-clamp" principle is that a) the device requires a servo system which is expensive and technically complex and cumbersome and b) the operating point needs frequent adjustment.

Devices for measuring the continuous arterial blood pressure of a finger are known, these devices are recording a volume change curve (for example a photoplethysmogram) and calculating a pressure curve from it.

Patent document US5,296,310, Jones et al., 14.12.1993 describes a method in which the systolic and diastolic pressure values for each cardiac cycle are obtained from the volume curve by multiplying the latter by a constant k. The method is inaccurate because the pressure and volume curves are not linearly related.

Patent documents Nos. US4,846,189, Sun Shuxing, 11.07.1989 and US5,423,322, Clark et al., 13.06.1995 assume that the relationship between pressure and volume curves is exponential. This gives a more accurate result in the calculations, but is still inaccurate, because the dependence of the function between the pressure and volume curves changes over time depending on the physiological condition of the person.

WO2017/152098 discloses a device for continuous non-invasive monitoring of arterial blood pressure based on the dependence function of pressure and volume curves for estimating arterial blood presseure comprising an optical sensor consisting of a light source and a photodetector, transimpedance amplifiers electrically connected to photodetectors, forces transducer attached to optical sensor, a microcontroller and an external communication port.

### Summary of invention

The present invention provides a method and apparatus for blood pressure measurement in the non-occlusive non-invasive continuous manner. The device comprises two optical, for example photoplethysmographic, sensors arranged side by side. The optical sensor consists of a light emitting diode and a photodiode that are placed next to each other at determined distance. The optical sensors are under two different applied pressures, which is realized with the cavity in the housing of the device. The surface of first optical sensor in relation to the second optical sensor is placed in the cavity. Both optical sensors are equipped with force transducer that measures the pressure that is applied by the optical sensor to the artery or microvascular bed of tissue. Alternatively, in order to produce differences in the back pressures exerted by the optical sensor, a spring is attached between the first optical sensor and the force transducer, the stiffness of which differs from that of the spring attached between the second optical sensor and the force transducer. The output voltage is in known relation with the applied force on the transducer. The LED of the optical sensor emits light that is absorbed and scattered in the artery or microvascular bed of tissue and fraction of photons are detected by photodiode. The detected pulsatile light intensity changes are related to the relative blood volume changes in the artery or microvascular bed of tissue. The photodiode signals from the optical sensors are connected to transimpedance amplifiers that convert the photocurrents of the photodiodes to the voltage signals. Voltage signals from the force transducers and transimpedance amplifiers are supplied to analogue-to-digital converter (ADC). The digital signals from ADC are supplied to microcontroller, where the volume difference signal amplitude $\Delta V_{12}$ or $\Delta V_{21}$ is calculated based on the signals from optical sensors. In addition, the cardiac cycles are detected and for each cycle the arterial compliance index k is calculated based on the relative blood volume change signals from the optical sensors and the pressures that are applied by the optical sensors. Memory is connected to the microcontroller, which is used to store the calibration parameter and signals during calibration manoeuvre. In addition, during the calibration manoeuvre the systolic and diastolic blood pressures are possible to supply to the microcontroller via external communication port, e.g. USB, Bluetooth etc., that is connected to microcontroller.

The above described device is firstly calibrated to determine certain parameter that is used by the microcontroller to continuously measure the systolic blood pressure (*SBP*), diastolic blood pressure (*DBP*), and pulse pressure (*PP*). There are two possible calibration manoeuvres. The first possible calibration manoeuvre includes the external device that determines the arterial blood pressure, e.g. oscillometric blood pressure device. The arterial blood pressure is measured by external blood pressure device and at the same time the calibration manoeuvre is initiated in the device via external communication port. During the calibration manoeuvre amplitudes $\Delta V_{12}$ or $\Delta V_{21}$ of the relative volume change differences, parameter k, and applied pressure signals are recorded to the memory. The recording is terminated in the microcontroller via external communication port after the blood pressure measurement is finished with the external device. As follows, the systolic blood pressure and diastolic blood pressure are supplied to the microcontroller via external communication port. The calibration parameter B is calculated based on the recorded data and blood pressure values.

The second possible calibration manoeuvre is initiated, when the microcontroller detects the rise in the force that is applied to the optical sensors or initiated via external communication port. The volume difference signal amplitude $\Delta V_{12}$ or $\Delta V_{21}$, arterial compliance index k, and applied pressure signal values are recorded to the memory for each cardiac cycle. The applied forces on the optical detectors can be monitored via external communication port. The applied pressure by the optical sensors is increased (e.g. manually with finger) and it exceeds the mean arterial blood pressure. Thereafter, the applied pressure is decreased back to the initial level, which is detected by the microcontroller, and the recording of the parameters to the memory is terminated automatically or via external communication port. The maximal values $\Delta V_{12\_max}$ or $\Delta V_{21\_max}$ of amplitudes $\Delta V_{12}$ or $\Delta V_{21}$ from the recorded time series is detected. Based on this point in the time series, the arterial compliance index $k_{max}$ and pressure sensor values $P_{s1\_max}$ and $P_{s2\_max}$ are detected and calibration parameter B is calculated.

The function (compliance model) between blood pressure and relative blood volume change is determined based on the calibration parameter B for particular patient and for every cardiac cycle updated compliance index k. The calculated systolic blood pressure, diastolic blood pressure, and pulse pressure values in the microcontroller are supplied via external communication port.

**Brief description of drawings**

The present invention will be described below in detailed description with reference to the accompanied drawings where:

Fig 1 shows the relationship between transmural pressure and blood volume in artery;

Fig 2 shows the relationship between transmural pressure and compliance of artery;

Fig 3 shows a blood volume change in artery in case mean transmural pressure is zero;

Fig 4 shows a blood volume changes in artery for two pressures sensors at different applied pressures;

Fig 5 shows a blood volume change in artery between two pressures sensors at different applied pressures;

Fig 6 illustrates a block diagram of a non-occlusive and continuous blood pressure sensor device, which is constructed according to the principles of current invention;

Fig 7 illustrates a construction principles of the blood pressure sensor device; a) is a cross section of the pressure sensor device, b) is a bottom view of the pressure sensor device;

Fig 8 illustrates a construction principles of an alternative solution of blood pressure sensor device; a) is a cross section of the pressure sensor device, b) is a bottom view of the pressure sensor device;

Fig 9 illustrates a flowchart of blood pressure monitoring and first calibration manoeuvre;

Fig 10 illustrates a flowchart of blood pressure monitoring and second calibration manoeuvre;

Fig 11 illustrates calculated volumes $\Delta V_1$, $\Delta V_2$, and $\Delta V_{21}$ ;

Fig 12 illustrates applied pressures on optical sensors;

Fig 13 to 16 illustrates results of estimated blood pressures using equations according to invention;

Fig 17 to 20 illustrates the Bland-Altman plots of the results illustrated in Fig 13 to 16.

**Detailed description of the invention**

The present invention provides for non-occlusive non-invasive continuous imposed arterial blood pressure monitoring. The systolic blood pressure, diastolic blood pressure and pulse pressure are obtained by calculation using arterial blood volume signals from two volume sensors, which are under two different applied pressures. The volume signals are obtained optically using optical sensing technique, which is widely known, and they represent the relative blood volume changes over time. The arterial blood pressure is estimated using the function, which relates the transmural pressure and compliance in the artery, and it is updated for each cardiac cycle. The function is based on the so-called compliance model, which has been discussed earlier in Baker, P.D., Westenskow, D.R. and Kück, K., "Theoretical analysis of non-invasive oscillometric maximum amplitude algorithm for estimating mean blood pressure", Med. Biol. Eng. Comput. 35, 1997, page 271-278.

Transmural pressure $P_t$ is the difference between the intra-arterial pressure $P$ and the externally applied pressure $P_s$ (e.g. applied by optical sensor). Transmural pressure is calculated as follows:

$$P_t = P - P_s \tag{1}$$

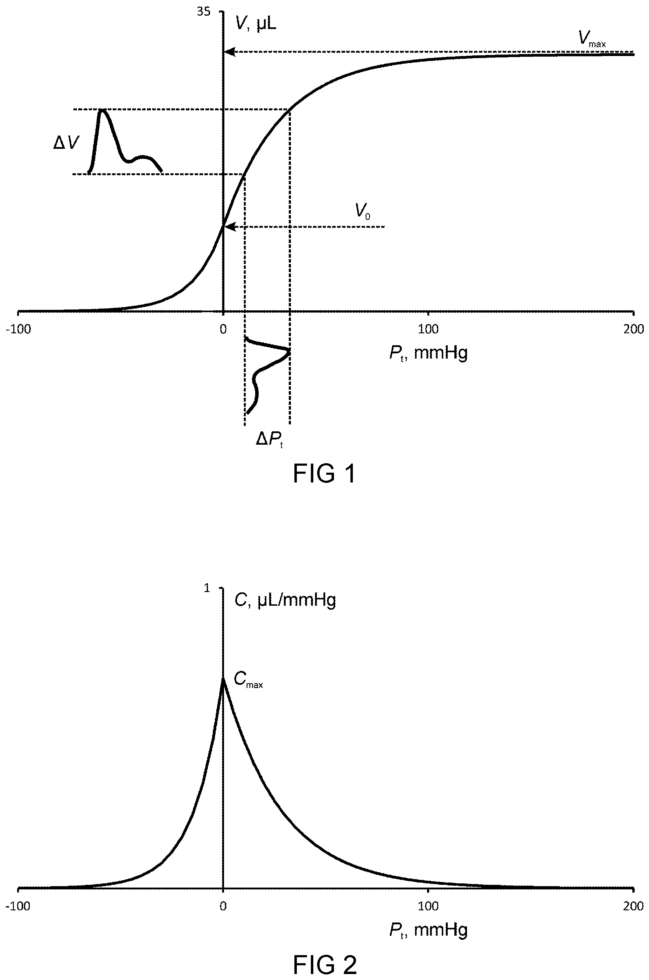

The blood volume V in artery and transmural pressure are related to each other through relationship, which is given in Fig 1. The blood volume in artery is given with the following equation, in case the Pt>0:

$$V = V_{\max} - (V_{\max} - V_0) \cdot e^{-\frac{c_m}{(V_{\max} - V_0)} \cdot P_t} \tag{2}$$

where $V_{\max}$ is the is the maximum arterial volume when the artery is fully expanded, $V_0$ is the arterial volume at zero $P_t$, and $C_m$ is the maximum compliance. It can be seen that even with the same change of transmural pressure $\Delta P_t$ the volume change $\Delta V$ is different depending on the operating point of $P_t$ (Fig 1). $\Delta V$ represents the relative volume change or amplitude within one cardiac cycle.

Through differentiation of equation 9 the analytical form can be obtained for the arterial compliance, in case $P_t$>0:

$$C = \frac{dV}{dP_t} \approx \frac{\Delta V}{\Delta P_t} = C_m \cdot e^{-\frac{c_m}{(V_{\max} - V_0)} \cdot P_t} \tag{3}$$

The relationship is illustrated in Fig 2.

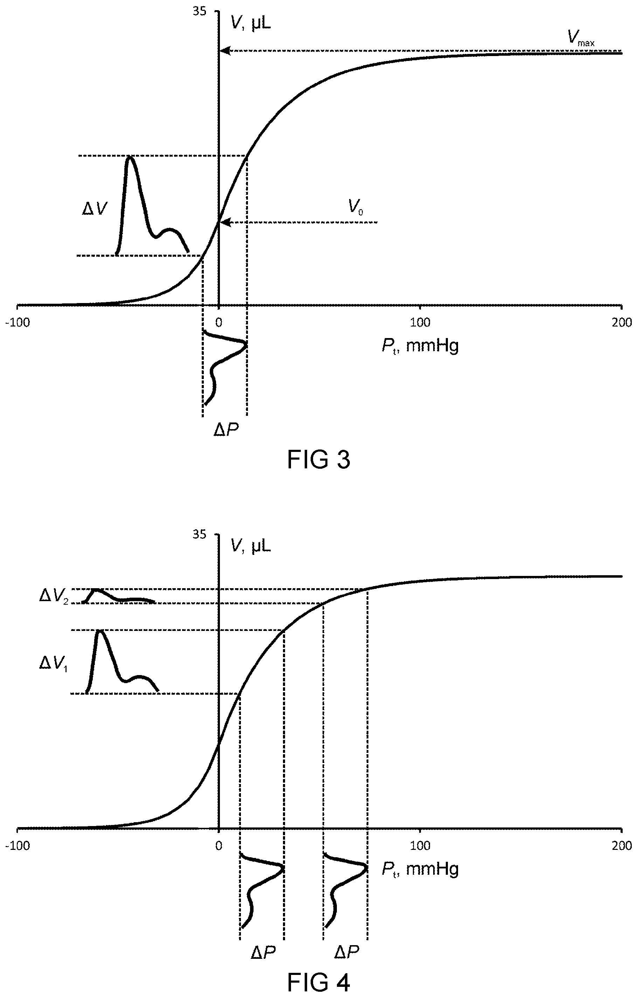

Blood volume change in artery is maximal in case mean transmural pressure is zero (see Fig 3). In such case the externally applied pressure is equal to the mean arterial pressure.

In the non-occlusive continuous (beat-to-beat) blood pressure estimation system the two blood volume sensors, S1 and S2, which are optical sensors in the present invention, are applied to the artery at two different pressures $P_{s1}$ and $P_{s2}$. In such case the blood pressure change $\Delta P$ in the artery is equal to the pulse pressure. For both blood volume sensors, the pulse pressure is the same; however, the blood volume changes under the sensor are different. The blood volume change for volume sensor with applied pressure $P_{s1}$ is equal to $\Delta V_1$ and for volume sensor with applied pressure $P_{s2}$ is equal to $\Delta V_2$.

For both volume sensors, the compliances of artery can be calculated as follows:

$$C_1 = \frac{\Delta V_1}{\Delta P}$$

$$C_2 = \frac{\Delta V_2}{\Delta P} \tag{4}$$

As pulse pressures are equal for both sensors (assuming that pulse pressure is not changing in such a short distance between two sensors) then from equation 4:

$$\frac{C_1}{C_2} = \frac{\Delta V_1}{\Delta V_2} \tag{5}$$

By substituting equation 3 to equation 5:

$$\frac{\Delta V_1}{\Delta V_2} = \frac{C_m \cdot e^{-\frac{C_m}{(V_{max}-V_0)} \cdot P_{t1}}}{C_m \cdot e^{-\frac{C_m}{(V_{max}-V_0)} \cdot P_{t2}}} = e^{-\frac{C_m}{(V_{max}-V_0)} \cdot (P_{t1}-P_{t2})} \qquad (6)$$

and

$$\frac{\ln\left(\frac{\Delta V_1}{\Delta V_2}\right)}{(P_{t1}-P_{t2})} = \frac{C_m}{(V_{max}-V_0)} = k. \qquad (7)$$

The equation 5 can be represented as well with opposite ratios:

$$\frac{C_2}{C_1} = \frac{\Delta V_2}{\Delta V_1}. \qquad (8)$$

By substituting equation 3 to equation 8:

$$\frac{\ln\left(\frac{\Delta V_2}{\Delta V_1}\right)}{(P_{t2}-P_{t1})} = \frac{C_m}{(V_{max}-V_0)} = k. \qquad (9)$$

The difference between transmural pressures of $P_{t1}$ and $P_{t2}$ ($P_{t1} < P_{t2}$) is equal to the difference between applied pressures of volume sensors $P_{s1}$ and $P_{s2}$ ($P_{s1} > P_{s2}$), which can be calculated as follows:

$$P_{t1} = P - P_{s1}, \qquad (10)$$

$$P_{t2} = P - P_{s2}, \qquad (11)$$

$$P_{t1} - P_{t2} = P - P_{s1} - P + P_{s2} = P_{s2} - P_{s1} \text{ and} \qquad (12)$$

$$P_{t2} - P_{t1} = P - P_{s2} - P + P_{s1} = P_{s1} - P_{s2}. \qquad (13)$$

Therefore, the equations 7 and 9 can be rewritten as follows:

$$\frac{\ln\left(\frac{\Delta V_1}{\Delta V_2}\right)}{(P_{s2}-P_{s1})} = \frac{C_m}{(V_{max}-V_0)} = k \text{ and} \qquad (14)$$

$$\frac{\ln\left(\frac{\Delta V_2}{\Delta V_1}\right)}{(P_{s1}-P_{s2})} = \frac{C_m}{(V_{max}-V_0)} = k. \qquad (15)$$

The compliance model in equation 3 can be rewritten based on the equations 14 and 15:

$$C = k \cdot (V_{max} - V_0) \cdot e^{-k \cdot P_t}. \qquad (16)$$

By knowing the difference between applied pressures of volume sensors and estimated relative blood volume changes the $k$ can be calculated using equations 14 or 15 and it is dependent on compliance of artery. It is known that the compliance of artery changes due to the slowly varying tonus of the muscles around the vessel driven by the nervous system. Therefore, the calculation of parameter k for each cardiac cycle updates the compliance model. It is assumed that the difference ($V_{max} - V_0$) is not changing because maximal volume of artery cannot increase or decrease (during short period of time the artery is not growing bigger) and can be estimated by individual calibration. Therefore, in the following text the difference ($V_{max}$ - $V_0$) is substituted by calibration parameter B.

The difference between transmural pressures is equal to the difference between applied pressures of volume sensors:

$$\Delta P_{s12} = P_{s1} - P_{s2} \quad \text{or} \quad (17)$$

$$\Delta P_{s21} = P_{s2} - P_{s1}. \quad (18)$$

The difference between applied pressures of volume sensors corresponds to the measured blood volume difference by volume sensor signals $V_1$ and $V_2$, and can be calculated as follows:

$$V_{12} = V_1 - V_2 \text{ or} \quad (19)$$

$$V_{21} = V_2 - V_1. \quad (20)$$

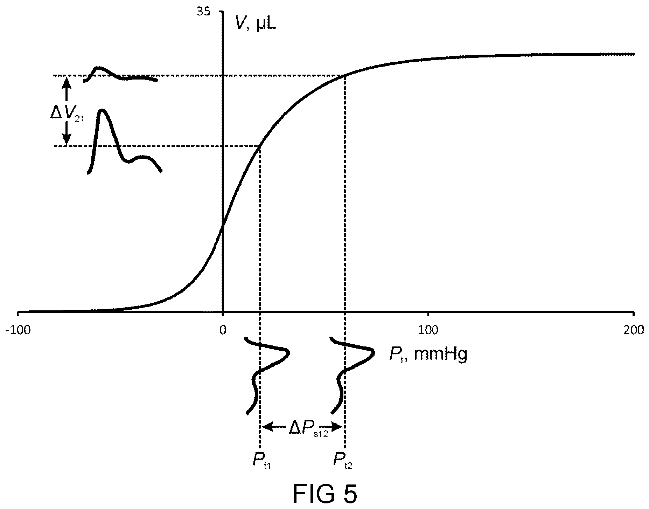

The amplitudes $\Delta V_{12}$ or $\Delta V_{21}$ of the volume difference signals $V_{12}$ or $V_{21}$ are detected for every cardiac cycle, respectively, and illustrated in Fig 5.

In such case, the compliance can be calculated based on equations 4 and 16 for the transmural pressure $P_{t1} + 0.5 \cdot \Delta P_{s12}$ as follows, in case Pt>0:

$$\frac{\Delta V_{21}}{\Delta P_{s12}} = k \cdot B \cdot e^{-k \cdot (P_{t1} + 0.5 \cdot \Delta P_{s12})}. \quad (21)$$

By substituting equation 1 into equation 21 it can be rewritten:

$$\frac{\Delta V_{21}}{\Delta P_{s12}} = k \cdot B \cdot e^{-k \cdot (P - P_{s1} + 0.5 \cdot \Delta P_{s12})}. \quad (22)$$

The intra-arterial pressure P derives from the equation 22 as follows:

$$P = P_{s1} - 0.5 \cdot \Delta P_{s12} - \frac{1}{k} \cdot \ln\left(\frac{\Delta V_{21}}{\Delta P_{s12} \cdot k \cdot B}\right). \quad (23)$$

Similarly, to the equation 21, the compliance model can be rewritten for the amplitude $\Delta V_{12}$:

$$\frac{\Delta V_{12}}{\Delta P_{s21}} = k \cdot B \cdot e^{-k \cdot (P_{t1} + 0.5 \cdot \Delta P_{s12})}. \quad (24)$$

In such case the amplitude $\Delta V_{12}$ and difference between applied pressures of volume sensors $\Delta P_{s21}$ are both negative. Based on the equation 1 and 24 the P derives similarly to the equation 23 as follows:

$$P = P_{s1} - 0.5 \cdot \Delta P_{s12} - \frac{1}{k} \cdot \ln\left(\frac{\Delta V_{12}}{\Delta P_{s21} \cdot k \cdot B}\right). \quad (25)$$

The P can be also derived from the equations 23 and 25 for the transmural pressure $P_{t2} - 0.5 \cdot \Delta P_{s12}$ as follows, in case Pt>0:

$$P = P_{s2} + 0.5 \cdot \Delta P_{s12} - \frac{1}{k} \cdot \ln\left(\frac{\Delta V_{21}}{\Delta P_{s12} \cdot k \cdot B}\right), \text{ and} \quad (26)$$

$$P = P_{s2} + 0.5 \cdot \Delta P_{s12} - \frac{1}{k} \cdot \ln\left(\frac{\Delta V_{12}}{\Delta P_{s21} \cdot k \cdot B}\right). \quad (27)$$

The equations 23, 25, 26, and 27 can be obtained respectively for the transmural pressures $P_{t1} - 0.5 \cdot \Delta P_{s21}$ and $P_{t2} + 0.5 \cdot \Delta P_{s21}$, in case Pt>0:

$$P = P_{s1} + 0.5 \cdot \Delta P_{s21} - \frac{1}{k} \cdot \ln\left(\frac{\Delta V_{21}}{\Delta P_{s12} \cdot k \cdot B}\right) \qquad (28)$$

$$P = P_{s1} + 0.5 \cdot \Delta P_{s21} - \frac{1}{k} \cdot \ln\left(\frac{\Delta V_{12}}{\Delta P_{s21} \cdot k \cdot B}\right) \qquad (29)$$

$$P = P_{s2} - 0.5 \cdot \Delta P_{s21} - \frac{1}{k} \cdot \ln\left(\frac{\Delta V_{21}}{\Delta P_{s12} \cdot k \cdot B}\right) \qquad (30)$$

$$P = P_{s2} - 0.5 \cdot \Delta P_{s21} - \frac{1}{k} \cdot \ln\left(\frac{\Delta V_{12}}{\Delta P_{s21} \cdot k \cdot B}\right) \qquad (31)$$

Intra-arterial pressure P can be estimated equally from equations 23, 25, 26, 27, 28, 29, 30, and 31, in case the calibration parameter B is determined through one point calibration. Therefore, the B is calculated in case the intra-arterial pressure P is known and it is derived from the equations 23 and 25:

$$B = \frac{\Delta V_{21}}{(P_{s1} - P_{s2}) \cdot k \cdot e^{-k \cdot (P - P_{s1} + 0.5 \cdot (P_{s1} - P_{s2}))}}, \qquad (32)$$

$$B = \frac{\Delta V_{12}}{(P_{s2} - P_{s1}) \cdot k \cdot e^{-k \cdot (P - P_{s1} + 0.5 \cdot (P_{s1} - P_{s2}))}}. \qquad (33)$$

Similarly, the calibration parameter B can be derived from all the intra-arterial pressure P equations 26 to 31. However, in the following text all the derivations are based on the equations 23 and 25. The intra-arterial pressure P can be determined using for example an external oscillometric blood pressure measurement device and the systolic blood pressure ($SBP_m$), diastolic blood pressure ($DBP_m$), mean blood pressure ($MBP_m$), and pulse pressure ($PP_m$) are measured. Any of previously mentioned two measured blood pressures can be selected for the intra-arterial pressure P calculation as they are all related to each other. However, here the intra-arterial pressure P is calculated using systolic and diastolic blood pressure and the calibration parameter B derives as follows based on the equations 32 and 33:

$$B = \frac{\Delta V_{21\_m}}{(P_{s1\_m} - P_{s2\_m}) \cdot k_m \cdot e^{-k_m \cdot \left(DBP_m + 0.5 \cdot (SBP_m - DBP_m) - P_{s1\_m} + 0.5 \cdot (P_{s1\_m} - P_{s2\_m})\right)}}, \text{ and } (34)$$

$$B = \frac{\Delta V_{12\_m}}{(P_{s2\_m} - P_{s1\_m}) \cdot k_m \cdot e^{-k_m \cdot \left(DBP_m + 0.5 \cdot (SBP_m - DBP_m) - P_{s1\_m} + 0.5 \cdot (P_{s1\_m} - P_{s2\_m})\right)}}, \qquad (35)$$

where $\Delta V_{21\_m}$, $k_m$, $P_{s1\_m}$, $P_{s2\_m}$ are the average values of parameters $\Delta V_{21}$, $\Delta V_{12}$, $k$, $P_{s1}$, $P_{s2}$ during the periode while blood pressure measurement was carried out by external device.

The calibration parameter B is derived as well in case the transmural pressure is zero $(P - P_{s1} + 0.5 \cdot \Delta P_{s12} = 0)$ in equation 22. In such case the amplitudes $\Delta V_{12}$ or $\Delta V_{21}$ of the volume difference signals are maximal $\Delta V_{12\_max}$ or $\Delta V_{21\_max}$. This situation is achieved by increasing the pressure, which is applied on volume sensors. The calibration parameter B is derived from the equation 22 for $\Delta V_{12\_max}$ or $\Delta V_{21\_max}$:

$$B = \frac{\Delta V_{21\_max}}{(P_{s1\_max} - P_{s2\_max}) \cdot k_{max}} \text{ or } \qquad (34)$$

$$B = \frac{\Delta V_{12\_max}}{(P_{s2\_max} - P_{s1\_max}) \cdot k_{max}}, \qquad (35)$$

where $k_{max}$, $P_{s1\_max}$ and $P_{s2\_max}$ are the values of $k$, $P_{s1}$, and $P_{s2}$ at the situation when $\Delta V_{12}$ or $\Delta V_{21}$ are maximal.

The compliance model is used for the intra-arterial pressure P calculations once the calibration parameter B is estimated. Based on the calculated intra-arterial pressure P, the pulse pressure (PP) is calculated by combining equations 4, 10, 11, and 16:

$$PP = \frac{\Delta V_1}{k \cdot B \cdot e^{-k \cdot (P - P_{s1})}} \quad , \quad (36)$$

$$PP = \frac{\Delta V_2}{k \cdot B \cdot e^{-k \cdot (P - P_{s2})}} \quad . \quad (37)$$

Systolic blood pressure is calculated based on equations 23, 36, and 37 as follows:

$$SBP = P + 0.5 \cdot PP. \quad (38)$$

Similarly, diastolic blood pressure is calculated based on equations 23, 36 and 37 as follows:

$$DBP = P - 0.5 \cdot PP. \quad (39)$$

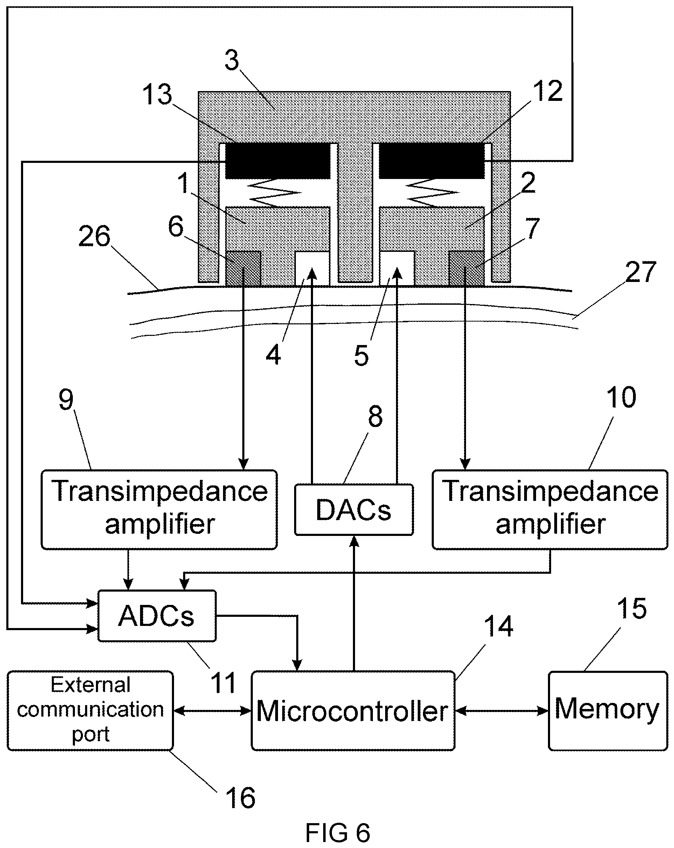

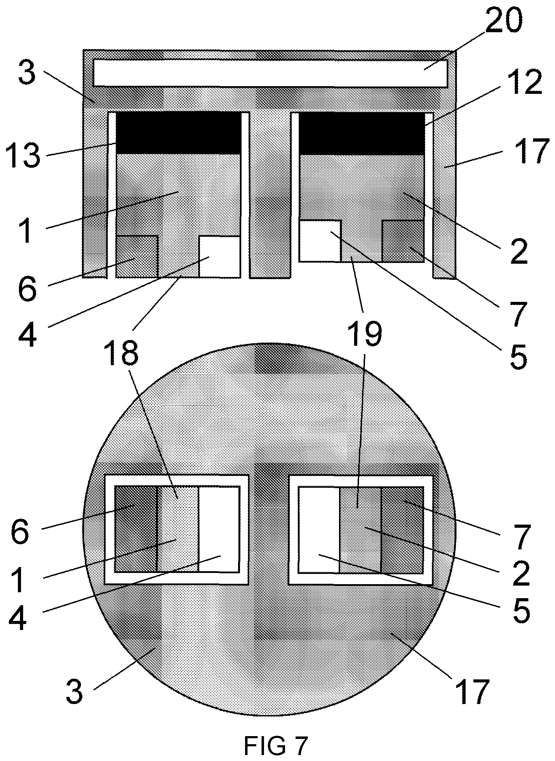

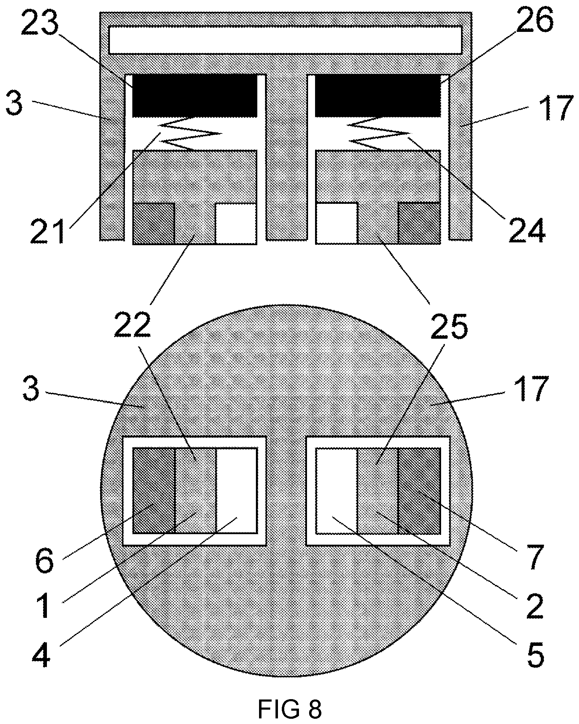

In the present invention, the device for non-occlusive non-invasive continuous pressure monitoring is shown in Fig 6. The dependence between the transmural pressure and compliance for each cardiac cycle is performed by updating the function (compliance model) adopted for sensor device. The sensor device comprises two pairs of photoplethysmographic sensors **1, 2** arranged side by side in one sensor device housing **3** as optical sensor comprising of a light source **4, 5** and a photodetector **6, 7**, digital-to-analogue converters (DACs) **8** electrically connected to light source, transimpedance amplifiers 9, 10 electrically connected to the photodetector, analogue-to-digital converters (ADCs) **11** electrically connected to the force transducers **12, 13** and the transimpedance amplifiers, a microcontroller **14** electrically connected to the analogue-to-digital and digital-to-analogue converters, an electrically connected memory **15** and external communication port **16** to the microcontroller. A force transducer is attached to each optical sensor. The back pressure exerted on the artery by both optical sensors can be measured with a force transducer. The sensor housing **17,** shown in Fig 7, comprises one or both optical sensors **18, 19** in the case of cavities. The sensor housing is designed so that the surfaces of the optical sensors are not in the same level. The surface of one optical sensor is located in relation to the surface of the other sensor in the cavity. Thereby, the optical sensor in the cavity exerts a lower back pressure than the sensor not in the cavity. The optical sensor and force transducer in the cavity is attached to the housing so that the arterial pressure exerted by the optical sensor can be recorded. The signals from the optical sensors and force transducers are supplied to the other electrical components of device **20**. In alternative embodiment, shown in Fig 8, in order to produce differences in the back pressures exerted by the optical sensors, a first spring **21** is attached between the first optical sensor **22** and the force transducer **23,** the stiffness of which differs from that of the second spring **24** attached between the second optical sensor **25** and the force transducer **26**.

The light from light emitting diodes (LEDs) is absorbed and scattered in the artery or microvascular bed of tissue and fraction of photons are detected by photodiode (photodetector). The current signal from photodiodes of optical sensors are supplied to transimpedance amplifiers that convert the photocurrents of the photodiodes to the voltage signals. The back pressure exerted on the artery by both optical sensors is measured with a force transducer. The output voltage of the transducer is in known relation with the applied force on the transducer. The outputs of the two transimpedance amplifiers and force transducers are supplied to the analogue-to-digital converters, where the signals are digitized for application to the microcontroller.

The microcontroller turns the LEDs on alternately through the DAC and the intensity of the LEDs are set based on the received voltage signals of photodetectors from the transimpedance amplifier. The driving frequency of the LEDs is at least 1kHz and the duty cycle is between 25% to 50%. The microcontroller assembles the light intensity signals based on the voltage signals received for each photodetector, while the LED is turned on. Microcontroller may cancel the ambient light by using the voltage signal while the LED is turned off and subtracting it from the signal while the LED is turned on. The relative volume signals $V_1$ and $V_2$ are computed using the principles of Beer-Lamber law:

$$I = I_0 \cdot e^{-\mu \cdot V} \quad , \quad (40)$$

where $I_0$ is emitted light intensity by LED, $I$ is detected light intensity by photodiode, V is tissue volume and $\mu$ is absorption.

In diastole, the arterial blood volume in tissue is minimal $V_{min}$ and the detected light intensity is maximal $I_{max}$. Beer-Lambert law is as follows:

$$I_{max} = I_0 \cdot e^{-\mu \cdot V_{min}} \qquad (41)$$

In systole, the arterial blood volume in tissue is maximal and the detected light intensity is minimal. For such case the Beer-Lambert law is as follows:

$$I_{min} = I_0 \cdot e^{-\mu \cdot V_{max}} \qquad . \qquad (42)$$

Therefore, the relative blood volume change in tissue is:

$$\frac{I_{max}}{I_{min}} = \frac{I_0 \cdot e^{-\mu \cdot V_{min}}}{I_0 \cdot e^{-\mu \cdot V_{max}}} = \frac{e^{-\mu \cdot V_{min}}}{e^{-\mu \cdot V_{max}}} = e^{-\mu \cdot V_{min} + \mu \cdot V_{max}}$$

$$ln\left(\frac{I_{min}}{I_{max}}\right) = -\mu \cdot V_{min} + \mu \cdot V_{max} = V_{max} - V_{min} = \Delta V. \quad (43)$$

Microcontroller detects for each cardiac cycle the minimal and maximal values of light intensities for both sensors and calculates volume changes $\Delta V_1$ and $\Delta V_2$ using the equation 43.

For the optical sensor S1 the relative blood volume can be calculated as follows:

$$I_1 = I_{01} \cdot e^{-\mu \cdot V_1} \qquad (44)$$

where $I_{01}$ is the emitted and $I_1$ is detected light intensity of optical sensor S1. Similarly, the light intensity can be calculated for the second optical sensor S2:

$$I_2 = I_{02} \cdot e^{-\mu \cdot V_2}. \qquad (45)$$

The difference between blood volumes underneath the sensors are calculated as follows:

$$\frac{I_1}{I_2} = \frac{I_{01} \cdot e^{-\mu \cdot V_1}}{I_{02} \cdot e^{-\mu \cdot V_2}} = \frac{I_1}{I_2} \cdot e^{-\mu \cdot V_1 + \mu \cdot V_2}$$

$$ln\left(\frac{I_1 \cdot I_{02}}{I_2 \cdot I_{01}}\right) = V_2 - V_1 = V_{21} \qquad \text{or} \qquad (46)$$

$$ln\left(\frac{I_2 \cdot I_{01}}{I_1 \cdot I_{02}}\right) = V_1 - V_2 = V_{12}. \qquad (47)$$

Microcontroller calculates according to the equation 46 or 47 the difference between blood volumes underneath the optical sensors and detects the amplitude $\Delta V_{21}$ or $\Delta V_{12}$ for each cardiac cycle, respectively. Furthermore, microcontroller calculates for each cardiac cycle pressures of the sensors $P_{s1}$ and $P_{s2}$ using the output voltages from force transducers, volume changes $\Delta V_1$ and $\Delta V_{21}$ parameter $k$ (compliance index), intra-arterial blood pressure P, pulse pressure $PP$, systolic blood pressure $SBP$, and diastolic blood pressure $DBP$, and supplies the values together with parameters $\Delta V_{21}$ or $\Delta V_{12}$ via external communication port.

During calibration procedure the microcontroller stores the parameters $\Delta V_{21}$ or $\Delta V_{12}$, $k$, $P_{s1}$, $P_{s2}$, for each cardiac cycle to the memory of the device. There is possibility to initiate and to terminate the calibration manoeuvre via external communication port. The parameter B is calculated and stored to the memory of the device after calibration manoeuvre by microcontroller.

Use of blood pressure monitoring device will now be described. The device is placed on surface of the skin **26** above the subject's artery **27** or microvascular bed of tissue under interest (Fig 6). An external force is applied to the device, which may be exerted, for example, by a strap attached around the device and the body to be examined. Firstly, the calibration parameter B is determined through calibration manoeuvre. There are two possible calibration manoeuvres.

The first possible calibration manoeuvre includes the external device that determines the arterial blood pressure, e.g. oscillometric blood pressure device. The arterial blood pressure is measured by external blood pressure device and at the same time the calibration manoeuvre is initiated in the device via external communication port. During the calibration manoeuvre amplitudes $\Delta V_{12}$ or $\Delta V_{21}$ of the relative volume change differences, parameter $k$, and applied pressure signals $P_{s1}$ and $P_{s2}$ are recorded to the memory. The recording is terminated in the microcontroller via external communication port after the blood pressure measurement is finished with the external device. As follows, the systolic blood pressure ($SBP_m$) and diastolic blood pressure ($DBP_m$) are supplied to the microcontroller via external communication port. The calibration parameter B is calculated based on the average values of the recorded parameters $\Delta V_{12}$ or $\Delta V_{21}$, parameter $k$, and applied pressure signals $P_{s1}$ and $P_{s2}$, and blood pressure values $SBP_m$ and $DBP_m$.

The second possible calibration manoeuvre is initiated, when the microcontroller detects the rise in the force that is applied to the optical sensors or initiated via external communication port. The volume difference signal amplitude $\Delta V_{12}$, arterial compliance index k, and applied pressure signal values $P_{s1}$ and $P_{s2}$ are recorded to the memory for each cardiac cycle. The applied forces on the optical detectors can be monitored via external communication port. The applied pressure by the optical sensors is increased (e.g. manually) and it exceeds the mean arterial blood pressure. Thereafter, the applied pressure is decreased back to the initial level, which is detected by the microcontroller, and the recording of the parameters to the memory is terminated automatically or via external communication port. The maximal values $\Delta V_{12\,max}$ or $\Delta V_{21\_max}$ of amplitudes $\Delta V_{12}$ or $\Delta V_{21}$ from the recorded time series is detected. Based on this point in the time series, the arterial compliance index $k_{max}$ and pressure sensor values $P_{s1\_max}$ and $P_{s2\_max}$ are detected and calibration parameter B is calculated.

Possible recalibration may be needed periodically depending on the time period that the device has been used continuously.

After calibration the function (compliance model) between blood pressure and relative blood volume change is determined based on the calibration parameter B for particular patient and for every cardiac cycle updated compliance index k. The calculated systolic blood pressure, diastolic blood pressure, and pulse pressure values in the microcontroller are supplied via external communication port.

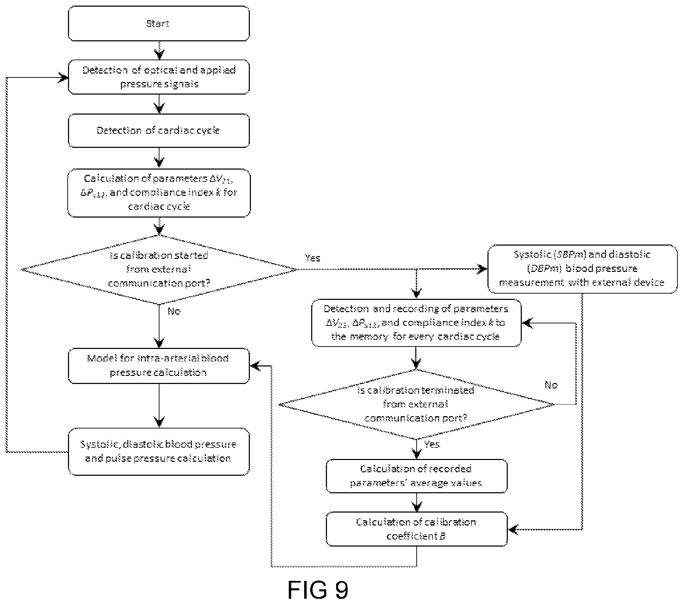

In Fig 9 is presented flowchart of the method according to present invention with first calibration manoeuvre where: device is attached to the individual and process starts with detection of optical and applied pressure signals, which is followed by detection of cardiac cycle, and based on obtained data parameters $\Delta V_{21}$, $P_{s12}$, and compliance index k are calculated, which is followed with systolic *(SBPm)* and diastolic *(DBPm)* blood pressure measurement with external device, and detection and recording of parameters $\Delta V_{21}$, $P_{s12}$, and compliance index $k$ in case calibration is started from external communication port, thereafter detection and recording of parameters is finished in case calibration is terminated from external communication port, which is followed with calculation of recorded parameters' average values and calibration coefficient B, in case calibration is not started or terminated from external communication port it is followed with intra-arterial blood pressure, systolic, diastolic and pulse pressure calculation.

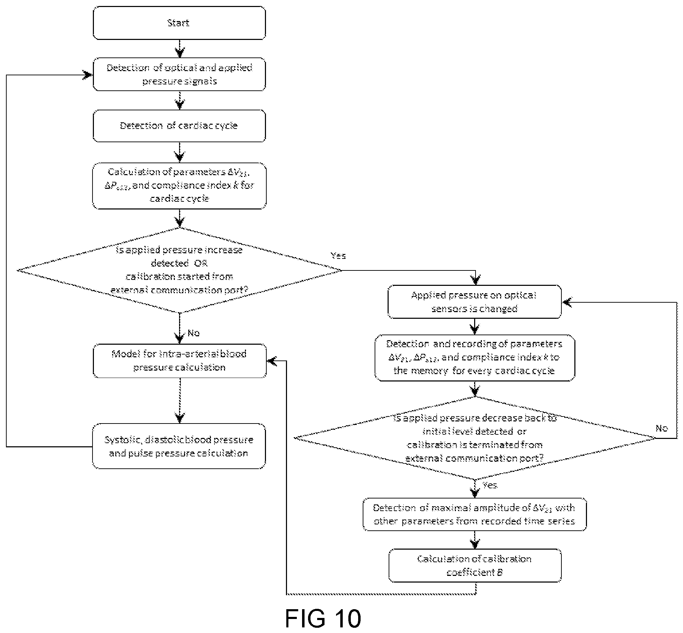

In Fig 10 is presented flowchart of the method according to present invention with second calibration manoeuvre where: device is attached to the individual and process starts with detection of optical and applied pressure signals, which is followed by detection of cardiac cycle, and based on obtained data parameters $\Delta V_{21}$, $P_{s12}$, and compliance index k are calculated, which is followed with applied pressure change on optical sensors and detection and recording of parameters $\Delta V_{21}$, $P_{s12}$, and compliance index k in case applied pressure increase on optical sensors is detected or calibration is started through external communication port, thereafter detection and recording of parameters is finished in case calibration is terminated from external communication port or applied pressure on optical sensors is returned to initial level, which is followed with detection of maximal amplitude of $\Delta V_{21}$ with other parameters from recorded time series and calculation of calibration coefficient B, in case calibration is not started or terminated from external communication port it is followed with intra-arterial blood pressure, systolic, diastolic and pulse pressure calculation.

It is to be understood that the above-described arrangements are only illustrative of the application of the principles of the present invention. Numerous modifications and alternative arrangements may be devised by those skilled in the art without departing from the spirit and scope of the present invention and the appended claims are intended to cover such modifications and arrangements. For example, sensing light transmitted through rather than back scattered from an artery or microvascular bed of tissue could be utilized to determine relative volume of the artery. Furthermore, any transducer, which converts blood volume or relative blood volume to electrical signal (e.g. bioimpedance), is applicable. The method is not limited with the blood volume measurement sites (e.g. radial artery etc.).

The method according to present invention was tested on three different subjects using two optical sensors, which were attached on the first finger. The applied pressures were different and lower than mean arterial pressure of the finger. The applied pressures were measured and recorded during the experiment. The Finapres system was used for the reference blood pressure measurement. The finger cuff was placed around middle finger. The optical signals were registered with sampling rate of 1kHz. During the experiment the subject was in supine position. The subjects were asked to carry out hand-grip test in order to change the arterial blood pressure during the recording time. After the recording of the signals the post processing was carried out in MATLAB.

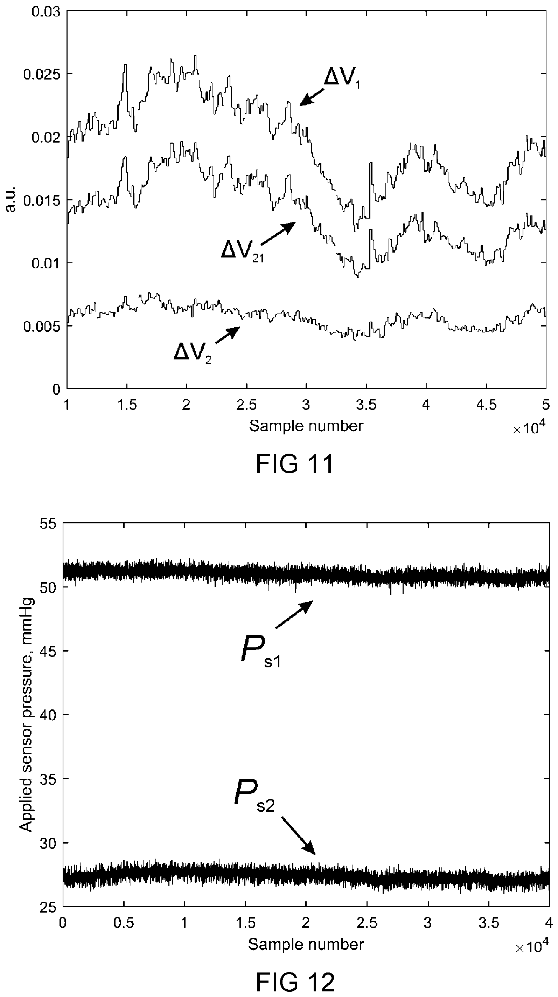

In Fig 11 are given volumes $\Delta V_1$, $\Delta V_{21}$ and $\Delta V_{21}$. The recorded pressure signals applied on the sensor are given in Fig 12. The calibration parameter B was determined using equation 32, according to the measured reference arterial systolic ($SBP_m$) and diastolic ($DBPm$) blood pressures.

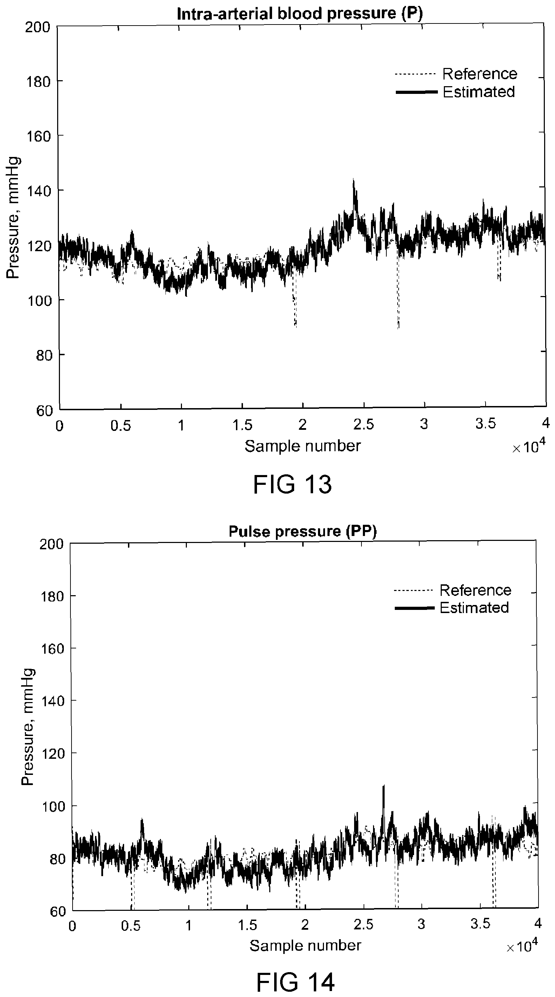

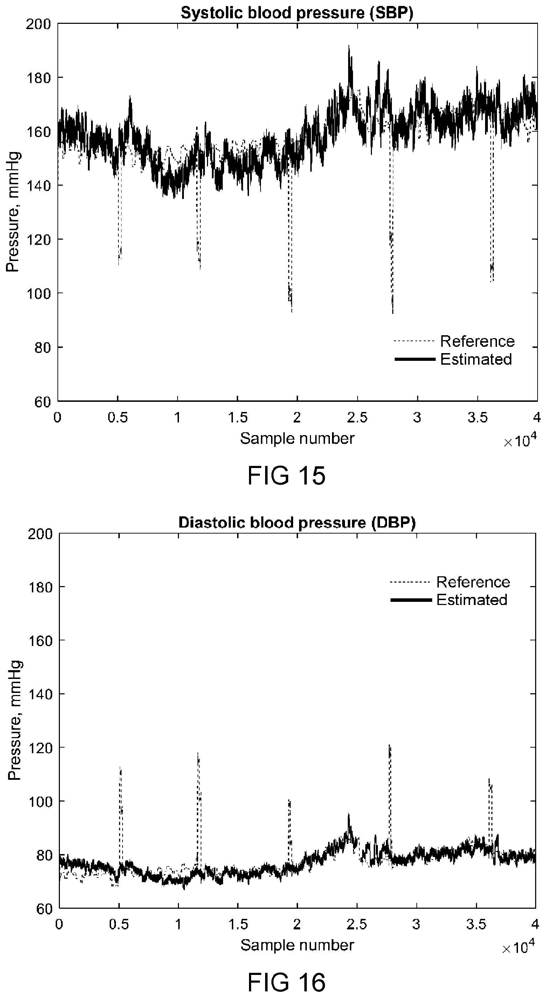

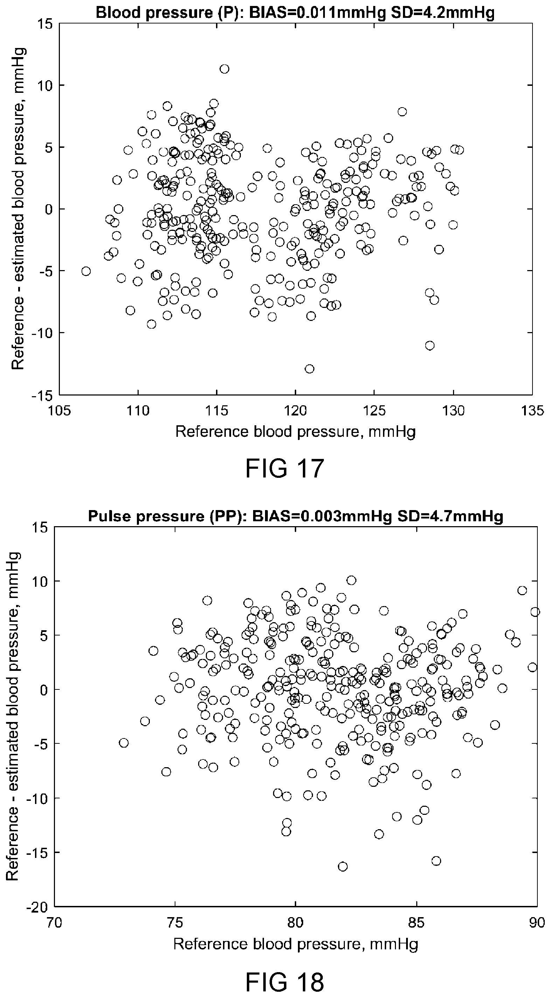

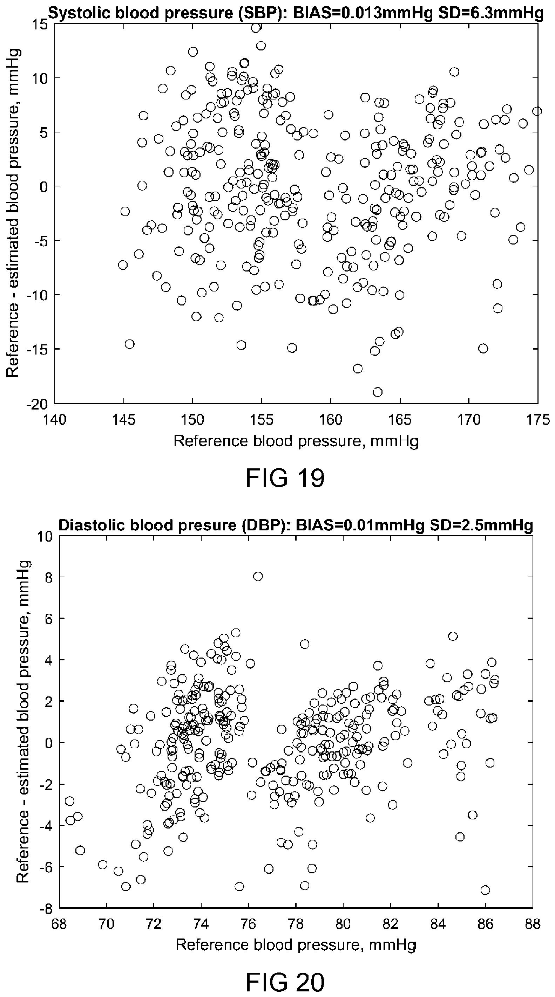

As follows, the blood pressures were estimated using equations 22, and 36 to 39. The results for first subject (subject nr. 1) are illustrated in Fig 13 to 16, where the Finapres measured and estimated blood pressures are given. The peaks can be observed from the Finapres measured blood pressure values. Those peaks are related to the sensor calibration of the Finapres device, which occurs after certain period of time. Those Finapres blood pressure values were excluded from the analysis. The Bland-Altman plots of the results are given in Fig 17 to 20. The bias (BIAS) and standard deviation (SD) values for each subject and blood pressure are given in Table 1.

Table 1. Bias and SD values of the estimated blood pressures for each subject.

|  |  | Subject nr. 1 | Subject nr. 2 | Subject nr. 3 |
|---|---|---|---|---|
| SBP | BIAS, mmHg | 0.013 | 0.026 | 0.874 |
|  | SD, mmHg | 6.3 | 6.2 | 6.7 |
| DBP | BIAS, mmHg | 0.01 | -0,024 | -1.368 |
|  | SD, mmHg | 2.5 | 2.4 | 5.5 |
| PP | BIAS, mmHg | 0.003 | 0.05 | 2.24 |
|  | SD, mmHg | 4.7 | 5.8 | 6.2 |
| P | BIAS, mmHg | 0.011 | 0.001 | -0.247 |
|  | SD, mmHg | 4.2 | 3.7 | 5.3 |

List of details

1, 2 - two pairs of photoplethysmographic sensors
3 - sensor device housing
4, 5 -light source
6, 7 - photodetector
8 - digital-to-analogue converters (DAC)
9, 10 - transimpedance amplifiers
11 - analogue-to-digital converters (ADCs)
12, 13 - force transducers
14 - microcontroller
15 - memory
16 - external communication port
17 - sensor housing
18, 19 - optical sensors
20 - electrical components of device
21 - first spring
22 - first optical sensor
23 - first force transducer
24 - second spring
25 - second optical sensor
26 - second force transducer
26 - subject skin
27 - subject artery

**Claims**

1. A method for continuous non-invasive monitoring of arterial blood pressure based on a beat-to-beat assessment of arterial blood pressure through a dependence function between the pressure and volume curves, **characterized by**

   - measuring, in time series, two volume curves by two volume sensors, each such volume sensor applying a back pressure to the artery, said back pressures being different from each other, and calculating the difference signal between said two volume curves by the formulas

$$V_{12} = V_1 - V_2,$$

or

$$V_{21} = V_2 - V_1,$$

where $V_1$ - a signal of the volume sensor with higher back pressure of said two volume sensors, $V_2$ - a signal of the volume sensor with lower back pressure of said two volume sensors, and
- amplitudes $\Delta V_{21}$ or $\Delta V_{12}$ of the difference signals $V_{12}$ or $V_{21}$ between the volume curves for each cardiac cycle are determined, and
- for each cardiac cycle, the arterial blood pressure with a predetermined calibration parameter is calculated from the amplitudes of the differential signal and the back pressures applied by the sensors by the formula

$$P = P_{s1} - 0.5 \cdot (P_{s1} - P_{s2}) - \frac{1}{k} \cdot \ln\left(\frac{\Delta V_{21}}{(P_{s1} - P_{s2}) \cdot k \cdot B}\right),$$

or by formula

$$P = P_{s1} - 0.5 \cdot (P_{s1} - P_{s2}) - \frac{1}{k} \cdot \ln\left(\frac{\Delta V_{12}}{(P_{s2} - P_{s1}) \cdot k \cdot B}\right),$$

or by formula

$$P = P_{s2} + 0.5 \cdot (P_{s1} - P_{s2}) - \frac{1}{k} \cdot \ln\left(\frac{\Delta V_{21}}{(P_{s1} - P_{s2}) \cdot k \cdot B}\right),$$

or by formula

$$P = P_{s2} + 0.5 \cdot (P_{s1} - P_{s2}) - \frac{1}{k} \cdot \ln\left(\frac{\Delta V_{12}}{(P_{s2} - P_{s1}) \cdot k \cdot B}\right),$$

where P - arterial blood pressure, $P_{s1}$ - value of higher back pressure applied by volume sensor for each cardiac cycle, $P_{s2}$ - value of lower back pressure applied by volume sensor for each cardiac cycle, k - compliance index determined for each cardiac cycle, B - parameter determined by previous individual calibration.

2. The method according to claim 1, **characterized in that** the dependence function between the pressure and volume curves is updated for each cardiac cycle by the compliance index k through the formula

$$k = \frac{\ln\left(\frac{\Delta V_1}{\Delta V_2}\right)}{(P_{s2} - P_{s1})},$$

or of the formula

$$k = \frac{\ln\left(\frac{\Delta V_2}{\Delta V_1}\right)}{(P_{s1} - P_{s2})},$$

where
$\Delta V_1$ - amplitude of the higher back pressure volume sensor signal determined for each cardiac cycle, $\Delta v_2$ - amplitude of the lower back pressure volume sensor signal for each cardiac cycle, $P_{s1}$ - value of the higher back pressure applied by the volume sensor for each cardiac cycle, $P_{s2}$ - value of the lower back pressure applied by the volume sensor for each cardiac cycle.

3. The method according to claim 1, **characterized in that** for each cardiac cycle the dependence function between the pressure and volume curves is updated and the pulse pressure is calculated by the formula

$$PP = \frac{\Delta V_1}{k \cdot B \cdot e^{-k \cdot (P - P_{s1})}},$$

or by the formula

$$PP = \frac{\Delta V_2}{k \cdot B \cdot e^{-k \cdot (P - P_{s2})}},$$

where k is compliance index determined for each cardiac cycle, B is parameter determined by previous individual calibration, $\Delta V_1$ - amplitude of the higher back pressure volume sensor signal determined for each cardiac cycle, $\Delta v_2$ - amplitude of the lower back pressure volume sensor signal for each cardiac cycle, $P_{s1}$ - value of the higher back pressure applied by the volume sensor for each cardiac cycle, $P_{s2}$ - value of the lower back pressure applied by the volume sensor for each cardiac cycle.

4. The method according to claims 1 and 3, **characterized in that** the systolic blood pressure for each cardiac cycle is calculated by the formula

$$SBP = P + 0.5 \cdot PP,$$

and the diastolic blood pressure by the formula

$$DBP = P - 0.5 \cdot PP$$

5. The method according to claim 1, **characterized in that** when determining arterial blood pressure for each cardiac cycle the applied pressures of volume sensors are lower than the mean arterial blood pressure.

6. The method according to claim 1, **characterized in that** for determining the individual calibration parameter B the pressure applied by volume sensors on the artery is increased above the mean arterial blood pressure while the difference of pressures applied by volume sensors maintained, during the increase of the back pressures the amplitude $\Delta V_{21}$ of the difference signal between the volume curves, compliance index k and time series of the back pressures $P_{s1}$, $P_{s2}$, are calculated, at the end of back pressures increase the maximum value $\Delta V_{21\_max}$ from the time series of the difference signal amplitudes $\Delta V_{21}$ between the volume curves and value of the compliance index $k_{max}$ corresponding to this time point and pressures $P_{s1\_max}$, $P_{s2\_max}$ applied by volume sensors are determined by using the formula

$$B = \frac{\Delta V_{21\_max}}{(P_{s1\_max} - P_{s2\_max}) \cdot k_{max}},$$

or formula

$$B = \frac{\Delta V_{12\_max}}{(P_{s2\_max} - P_{s1\_max}) \cdot k_{max}}.$$

7. The method according to claim 1, **characterized in that** for determining the individual calibration parameter B the arterial systolic blood pressure ( $SBP_m$ ) and diastolic blood pressure ( $DBP_m$ ) are measured by external blood pressure device and simultaneously with the measurement the time series of the parameters $\Delta V_{21}$ or $\Delta V_{12}$, k, $P_{s1}$, $P_{s2}$, are calculated and after measurement of the blood pressure the mean values of the time series of the parameters $\Delta V_{21\_m}$ or $\Delta V_{12\_m}$, $k_m$, $P_{s1\_m}$, $P_{s2\_m}$ are calculated by using the formula

$$B = \frac{\Delta V_{21\_m}}{(P_{s1\_m} - P_{s2\_m}) \cdot k_m \cdot e^{-k_m \cdot (DBP_m + 0.5 \cdot (SBP_m - DBP_m) - P_{s1\_m} + 0.5 \cdot (P_{s1\_m} - P_{s2\_m}))}},$$

or formula

$$B = \frac{\Delta V_{12\_m}}{(P_{s2\_m} - P_{s1\_m}) \cdot k_m \cdot e^{-k_m \cdot (DBP_m + 0.5 \cdot (SBP_m - DBP_m) - P_{s1\_m} + 0.5 \cdot (P_{s1\_m} - P_{s2\_m}))}}.$$

8. Device for continuous non-invasive monitoring of arterial blood pressure based on the dependence function of pressure and volume curves for estimating arterial blood pressure comprising two optical sensors (1, 2) consisting of a light source (4, 5) and a photodetector (6, 7), digital-analogue converters (8) attached to the light sources, transimpedance amplifiers (9, 10) electrically connected to photodetectors, force transducers (12, 13) attached to optical sensors, analogue-to-digital converters (11) electrically connected to force transducers and transimpedance amplifiers, a microcontroller (14) electrically connected to the analogue-to-digital converters and digital-to-analogue converters, a memory (15) electrically connected to the microcontroller and an external communication port (16), **characterized in that**
the sensor housing (17) comprises recesses for one or both optocouples in order to produce differences in the back pressures exerted by the optical sensors.

9. The device according to claim 8, wherein said force transducer is spring loaded wherein a first spring (21) is mounted between the first optical sensor (22) and the first force transducers (23), the stiffness of which differs 0.1 to 2 times from the stiffness of the second spring mounted between the second optical sensor (25) and the second force transducers (26), in order to create differences in the back pressures expressed by the optical sensors (1, 2).

10. The device according to claim 8 or 9, **characterised in that** the difference signal $V_{12}$ or $V_{21}$ between the volume curves and amplitude $\Delta V_{12}$ or $\Delta V_{21}$ is calculated in the microcontroller for the determination of arterial blood pressure.

11. The device according to claim 10, **characterized in that** the compliance index k of the function between pressure and volume curves is calculated in the microcontroller for each cardiac cycle.

12. The device according to claim 8 or 9, **characterized in that** the device is automatically switched to calibration mode when an increase in the pressures measured by force transducers is detected or device is switched to the calibration mode through external port, and in which the difference signal amplitude $\Delta V_{12}$ or $\Delta V_{12}$, compliance index k and back pressures $P_{s1}$ and $P_{s2}$ are stored in the memory attached to the controller for each cardiac cycle and simultaneously their values are sent out through external communication port.

13. The device according to claim 12, **characterized in that** the device detects a drop of pressures close to the initial level following an increase in the pressures measured by force transducers, as a result of which the recording of parameters ends or recording is terminated via the external communication port and from the time series of amplitudes $\Delta V_{12}$ the maximum amplitude $\Delta V_{12\_max}$ and corresponding compliance index k value $k_{max}$ and values of pressures $P_{s1\_max}$ ja $P_{s2\_max}$ applied by volume sensors are determined and the calibration parameter is calculated.

14. The device according to claim 8 or 9, **characterized in that** the device is switched to the calibration mode via the external communication port and during which parameters $\Delta V_{21}$ or $\Delta V_{12}$, k, $P_{s1}$, $P_{s2}$ for each cardiac cycle are stored in the memory attached to the microcontroller and that the device is switched off from calibration mode via the external communication port and the systolic ( SBPm ) and diastolic ( DBPm ) blood pressure values measured with an external blood pressure device are entered through the said port and based on the time series of the parameters stored in the memory the microcontroller calculates the mean values $\Delta V_{21\_m}$ $\Delta V_{12\_m}$, $k_m$, $P_{s1\_m}$. $P_{s2\_m}$ after the end of the blood pressure measurement and calculates the calibration parameter B.

15. Device according to claim 14, **characterized in that** the arterial blood pressure P, pulse pressure PP, systolic blood pressure SBP and diastolic blood pressure DBP are calculated for each heart cycle in the microcontroller of the device and these values are output via the communication port, respectively.

**Patentansprüche**

1. Verfahren zur kontinuierlichen, nicht-invasiven Überwachung des arteriellen Blutdrucks anhand einer Schlag-für-Schlag-Bewertung des Blutdrucks durch eine Abhängigkeitsfunktion zwischen Druck- und Volumenkurven, **dadurch gekennzeichnet, dass**

- Messung von zwei Volumenkurven in einer Zeitreihe mittels zweier Volumensensoren, wobei jeder dieser Volumensensoren einen Rückdruck auf die Arterie ausübt, wobei sich diese Rückdrücke voneinander unterscheiden, und Berechnung des Differenzsignals zwischen diesen beiden Volumenkurven nach den Formeln

$$V_{12} = V_1 - V_2, \text{ oder}$$

$$V_{21} = V_2 - V_1, \text{ wobei}$$

$V_1$ - Signal des Volumensensors mit dem höheren Rückdruck von den beiden genannten Volumensensoren, $V_2$ - Signal des Volumensensors mit dem niedrigeren Rückdruck von den beiden genannten Volumensensoren, und
- es werden die Amplituden $\Delta V_{21}$ oder $\Delta V_{12}$ der Differenzsignale $V_{12}$ bzw. $V_{21}$ zwischen den Volumenkurven jeweils für jeden Herzzyklus bestimmt, und
- für jeden Herzzyklus wird der arterielle Blutdruck mit einem vorbestimmten Kalibrierparameter aus den Amplituden des Differenzsignals und den von den Sensoren angelegten Rückdrücken gemäß der Formel berechnet

$$P = P_{s1} - 0.5 \cdot (P_{s1} - P_{s2}) - \frac{1}{k} \cdot \ln\left(\frac{\Delta V_{21}}{(P_{s1} - P_{s2}) \cdot k \cdot B}\right),$$

oder gemäß der Formel

$$P = P_{s1} - 0.5 \cdot (P_{s1} - P_{s2}) - \frac{1}{k} \cdot \ln\left(\frac{\Delta V_{12}}{(P_{s2} - P_{s1}) \cdot k \cdot B}\right),$$

oder gemäß der Formel

$$P = P_{s2} + 0.5 \cdot (P_{s1} - P_{s2}) - \frac{1}{k} \cdot \ln\left(\frac{\Delta V_{21}}{(P_{s1} - P_{s2}) \cdot k \cdot B}\right),$$

oder gemäß der Formel

$$P = P_{s2} + 0.5 \cdot (P_{s1} - P_{s2}) - \frac{1}{k} \cdot \ln\left(\frac{\Delta V_{12}}{(P_{s2} - P_{s1}) \cdot k \cdot B}\right),$$

wobei P - der arterielle Blutdruck, $Ps_1$ - der höhere Rückdruckwert, der vom Volumensensor für jeden Herzzyklus angelegt, $Ps_2$ - der niedrigere Rückdruckwert, der vom Volumensensor für jeden Herzzyklus angelegt, k - der Compliance-Index, der für jeden Herzzyklus bestimmt, B - ein Parameter, der durch eine vorherige individuelle Kalibrierung festgelegt.

2. Verfahren nach Anspruch 1, **dadurch gekennzeichnet, dass** die Abhängigkeitsfunktion zwischen Druck- und Volumenkurven für jeden Herzzyklus durch den Compliance-Index k gemäß der Formel aktualisiert wird

$$k = \frac{\ln\left(\frac{\Delta V_1}{\Delta V_2}\right)}{(P_{s2} - P_{s1})},$$

oder gemäß der Formel

$$k = \frac{\ln\left(\frac{\Delta V_2}{\Delta V_1}\right)}{(P_{s1} - P_{s2})},$$

wobei

$\Delta V_1$ - Amplitude des Signals des Volumensensors mit höherem Rückdruck, bestimmt für jeden Herzzyklus, $\Delta V_2$ - Amplitude des Signals des Volumensensors mit niedrigerem Rückdruck für jeden Herzzyklus, $P_{s1}$ - Wert des höheren Rückdrucks, der vom Volumensensor für jeden Herzzyklus angelegt, $P_{s2}$ - Wert des niedrigeren Rückdrucks, der vom Volumensensor für jeden Herzzyklus angelegt.

3. Verfahren nach Anspruch 1, **dadurch gekennzeichnet, dass** für jeden Herzzyklus die Abhängigkeitsfunktion zwischen den Druck- und Volumenkurven aktualisiert wird und der Pulsdruck nach folgender Formel berechnet wird

$$PP = \frac{\Delta V_1}{k \cdot B \cdot e^{-k \cdot (P - P_{s1})}},$$

oder gemäß der Formel

$$PP = \frac{\Delta V_2}{k \cdot B \cdot e^{-k \cdot (P - P_{s2})}},$$

wobei k der für jeden Herzzyklus bestimmte Compliance-Index ist, B ein durch eine vorhergehende individuelle Kalibrierung festgelegter Parameter, $\Delta V_1$ - die Amplitude des Signals des Volumensensors mit höherem Rückdruck, bestimmt für jeden Herzzyklus, $\Delta V_2$ - die Amplitude des Signals des Volumensensors mit niedrigerem Rückdruck für jeden Herzzyklus, $P_{s1}$ - der höhere Rückdruckwert, der vom Volumensensor für jeden Herzzyklus angelegt, $P_{s2}$ - der niedrigere Rückdruckwert, der vom Volumensensor für jeden Herzzyklus angelegt.

4. Verfahren nach den Ansprüchen 1 und 3, **dadurch gekennzeichnet, dass** für jeden Herzzyklus der systolische Blutdruck gemäß der Formel

$$SBP = P + 0.5 \cdot PP,$$

und der diastolische Blutdruck gemäß der Formel

$$DBP = P - 0.5 \cdot PP$$

berechnet wird.

5. Verfahren nach Anspruch 1, **dadurch gekennzeichnet, dass** bei der Bestimmung des arteriellen Blutdrucks für jeden Herzzyklus die von den Volumensensoren angelegten Drücke unterhalb des mittleren arteriellen Blutdrucks liegen.

6. Verfahren nach Anspruch 1, **dadurch gekennzeichnet, dass** zur Bestimmung des individuellen Kalibrierparameters B der von den Volumensensoren auf die Arterie ausgeübte Druck über den mittleren arteriellen Blutdruck erhöht wird, wobei die Druckdifferenz zwischen den Sensoren beibehalten wird, während dieser Druckerhöhung die Amplitude $\Delta V_{21}$ des Differenzsignals zwischen den Volumenkurven, der Compliance-Index k sowie die Zeitreihe der Rück-

drücke $P_{s1}$ und $P_{s2}$ berechnet werden, am Ende der Druckerhöhung aus dieser Zeitreihe der maximale Wert $\Delta V_{21\text{-}max}$ und der zugehörige Compliance-Index $k_{max}$ sowie die Drücke $P_{s1\text{-}max}$ und $P_{s2\text{-}max}$ bestimmt werden und gemäß der folgenden Formel berechnet wird

$$B = \frac{\Delta V_{21\_\text{max}}}{\left(P_{s1\_\text{max}} - P_{s2\_\text{max}}\right) \cdot k_{\text{max}}},$$

oder gemäß der Formel

$$B = \frac{\Delta V_{12\_\text{max}}}{\left(P_{s2\_\text{max}} - P_{s1\_\text{max}}\right) \cdot k_{\text{max}}}.$$

7. Verfahren nach Anspruch 1, **dadurch gekennzeichnet, dass** zur Bestimmung des individuellen Kalibrierparameters B der arterielle systolische Blutdruck ($SBP_m$) und diastolische Blutdruck mittels eines externen Blutdruckmessgeräts ermittelt werden und gleichzeitig die Zeitreihen der Parameter $\Delta V_{21}$ oder $\Delta V_{12}$, k, $P_{s1}$, $P_{s2}$ berechnet werden und nach Abschluss der Blutdruckmessung aus den Zeitreihen der Mittelwerte $\Delta V_{21\text{-}m}$ oder $\Delta V_{12\text{-}m}$, $k_m$, $P_{s1\text{-}m}$, $P_{s2\text{-}m}$ gebildet und gemäß der folgenden Formel berechnet wird

$$B = \frac{\Delta V_{21\_\text{m}}}{\left(P_{s1\_\text{m}} - P_{s2\_\text{m}}\right) \cdot k_{\text{m}} \cdot e^{-k_{\text{m}} \cdot \left(DBP_{\text{m}} + 0.5 \cdot (SBP_{\text{m}} - DBP_m) - P_{s1\_\text{m}} + 0.5 \cdot (P_{s1\_\text{m}} - P_{s2\_\text{m}})\right)}},$$

oder gemäß der Formel

$$B = \frac{\Delta V_{12\_\text{m}}}{\left(P_{s2\_\text{m}} - P_{s1\_\text{m}}\right) \cdot k_{\text{m}} \cdot e^{-k_{\text{m}} \cdot \left(DBP_{\text{m}} + 0.5 \cdot (SBP_{\text{m}} - DBP_m) - P_{s1\_\text{m}} + 0.5 \cdot (P_{s1\_\text{m}} - P_{s2\_\text{m}})\right)}}.$$

8. Vorrichtung zur kontinuierlichen, nicht-invasiven Überwachung des arteriellen Blutdrucks basierend auf einer Abhängigkeitsfunktion zwischen Druck- und Volumenkurven zur Schätzung des arteriellen Blutdrucks, umfassend zwei optische Sensoren (1, 2) mit einer Lichtquelle (4, 5) und einem Photodetektor (6, 7), Digital-Analog-Wandler (8) an den Lichtquellen, Transimpedanzverstärker (9, 10) für die Photodetektoren, Kraftaufnehmer (12, 13) an den optischen Sensoren, Analog-Digital-Wandler (11) für die Kraftaufnehmer und Transimpedanzverstärker, einen Mikrocontroller (14) elektrisch verbunden mit den Analog-Digital- und Digital-Analog-Wandlern, einen Speicher (15) elektrisch verbunden mit dem Mikrocontroller und eine externe Kommunikationsschnittstelle (16), **dadurch gekennzeichnet, dass** das Sensorgehäuse (17) Ausnehmungen für einen oder beide Optokoppler aufweist, um Unterschiede in den von den optischen Sensoren ausgeübten Rückdrücken zu erzeugen.

9. Vorrichtung nach Anspruch 8, wobei der Kraftaufnehmer federbelastet ist, wobei zwischen dem ersten optischen Sensor (22) und dem ersten Kraftaufnehmer (23) eine erste Feder (21) angeordnet ist, deren Steifigkeit sich um den Faktor 0.1 bis 2 von der Steifigkeit der zweiten Feder unterscheidet, welche zwischen dem zweiten optischen Sensor (25) und dem zweiten Kraftaufnehmer (26) angeordnet ist, um Unterschiede in den von den optischen Sensoren (1, 2) ausgeübten Rückdrücken zu erzeugen.

10. Vorrichtung nach Anspruch 8 oder 9, **dadurch gekennzeichnet, dass** das Differenzsignal $V_{12}$ oder $V_{21}$ zwischen den Volumenkurven und dessen Amplitude $\Delta V_{12}$ oder $\Delta V_{21}$ im Mikrocontroller zur Ermittlung des arteriellen Blutdrucks berechnet werden.

11. Vorrichtung nach Anspruch 10, **dadurch gekennzeichnet, dass** der Compliance-Index k der Funktion zwischen Druck- und Volumenkurven im Mikrocontroller für jeden Herzzyklus berechnet wird.

12. Vorrichtung nach Anspruch 8 oder 9, **dadurch gekennzeichnet, dass** die Vorrichtung automatisch in einen Kalibrierungsmodus wechselt, wenn ein Anstieg der von den Kraftaufnehmern gemessenen Drücke erkannt wird oder dass die Vorrichtung über eine externe Schnittstelle in den Kalibrierungsmodus versetzt wird, und dass dabei für jeden Herzzyklus die Differenzsignalamplitude $\Delta V_{12}$ oder $\Delta V_{12}$, der Compliance-Index k sowie die Rückdrücke $P_{s1}$ und $P_{s2}$ im dem Mikrocontroller zugeordneten Speicher abgelegt und gleichzeitig deren Werte über die externe Kommunikationsschnittstelle ausgegeben werden.

**13.** Vorrichtung nach Anspruch 12, **dadurch gekennzeichnet, dass** die Vorrichtung nach einer während des Anstiegs der von den Kraftaufnehmern gemessenen Drücke erkannten Rückkehr des Drucks auf ein dem Ausgangszustand nahekommendes Niveau den Parameterauszeichnungsvorgang beendet oder über die externe Kommunikationsschnittstelle beendet wird und dass aus der Zeitreihe der Differenzsignalamplituden $\Delta V_{12}$ der maximale Wert $\Delta V_{12\text{-}max}$ sowie der zugehörige Compliance-Index $k_{max}$ und die von den Volumensensoren angelegten Druckwerte $P_{s1\text{-}max}$ und $P_{s2\text{-}max}$ bestimmt und daraus der Kalibrierparameter berechnet wird.

**14.** Vorrichtung nach Anspruch 8 oder 9, **dadurch gekennzeichnet, dass** die Vorrichtung über die externe Kommunikationsschnittstelle in den Kalibrierungsmodus versetzt wird, währenddessen die Parameter $\Delta V_{21}$ oder $\Delta V_{12}$, k, $P_{s1}$ und $P_{s2}$ für jeden Herzzyklus im dem Mikrocontroller zugeordneten Speicher abgelegt werden, und dass die Vorrichtung nach dem Kalibrierungsmodus über die externe Kommunikationsschnittstelle ausgeschaltet wird, die mittels eines externen Blutdruckmessgeräts gemessenen systolischen ($SBP_m$) und diastolischen Blutdruckwerte eingegeben werden und auf der Grundlage der im Speicher gespeicherten Zeitreihen die Mittelwerte $\Delta V_{21\text{-}m}$, $\Delta V_{12\text{-}m}$, $k_m$, $P_{s1\text{-}m}$, $P_{s2\text{-}m}$ berechnet und der Kalibrierparameter B bestimmt wird.

**15.** Vorrichtung nach Anspruch 14, **dadurch gekennzeichnet, dass** der arterielle Blutdruck P, der Pulsdruck PP, der systolische Blutdruck SBP und der diastolische Blutdruck DBP für jeden Herzzyklus im Mikrocontroller der Vorrichtung berechnet und diese Werte jeweils über die Kommunikationsschnittstelle ausgegeben werden.

**Revendications**

**1.** Procédé de surveillance continue et non invasive de la pression artérielle, basé sur une évaluation battement par battement de la pression artérielle à l'aide d'une fonction de dépendance entre les courbes de pression et de volume, **caractérisé en ce que**

- mesurant, sous forme de séries temporelles, deux courbes de volume à l'aide de deux capteurs de volume, chacun de ces capteurs appliquant une contre-pression à l'artère, ces contre-pressions étant différentes l'une de l'autre, et en calculant le signal différentiel entre lesdites deux courbes de volume selon les formules

$$V_{12} = V_1 - V_2, \text{ ou}$$

$$V_{21} = V_2 - V_1, \text{ où}$$

$V_1$ - signal du capteur de volume appliquant la contre-pression la plus élevée parmi les deux capteurs de volume, $V_2$ - signal du capteur de volume appliquant la contre-pression la plus basse parmi les deux capteurs de volume, et
- les amplitudes $\Delta V_{21}$ ou $\Delta V_{12}$ des signaux différentiels $V_{12}$ ou $V_{21}$ entre les courbes de volume sont déterminées pour chaque cycle cardiaque, et
- pour chaque cycle cardiaque, la pression artérielle est calculée à partir des amplitudes du signal différentiel et des contre-pressions appliquées par les capteurs, à l'aide d'un paramètre d'étalonnage prédéterminé, selon la formule

$$P = P_{s1} - 0.5 \cdot (P_{s1} - P_{s2}) - \frac{1}{k} \cdot \ln\left(\frac{\Delta V_{21}}{(P_{s1}-P_{s2})\cdot k \cdot B}\right),$$

ou selon la formule

$$P = P_{s1} - 0.5 \cdot (P_{s1} - P_{s2}) - \frac{1}{k} \cdot \ln\left(\frac{\Delta V_{12}}{(P_{s2}-P_{s1})\cdot k \cdot B}\right),$$

ou selon la formule

$$P = P_{s2} + 0.5 \cdot (P_{s1} - P_{s2}) - \frac{1}{k} \cdot \ln\left(\frac{\Delta V_{21}}{(P_{s1}-P_{s2})\cdot k \cdot B}\right),$$

ou selon la formule

$$P = P_{s2} + 0.5 \cdot (P_{s1} - P_{s2}) - \frac{1}{k} \cdot \ln\left(\frac{\Delta V_{12}}{(P_{s2} - P_{s1}) \cdot k \cdot B}\right),$$

où P - la pression artérielle, $P_{s1}$ - la valeur de la contre-pression supérieure appliquée par le capteur de volume pour chaque cycle cardiaque, $P_{s2}$ - la valeur de la contre-pression inférieure appliquée par le capteur de volume pour chaque cycle cardiaque, k - l'indice de compliance déterminé pour chaque cycle cardiaque, B - le paramètre déterminé lors d'une étalonnage individuel préalable.

2. Procédé selon la revendication 1, **caractérisé en ce que** la fonction de dépendance entre les courbes de pression et de volume est mise à jour pour chaque cycle cardiaque par l'indice de compliance k selon la formule

$$k = \frac{\ln\left(\frac{\Delta V_1}{\Delta V_2}\right)}{(P_{s2} - P_{s1})},$$

ou selon la formule

$$k = \frac{\ln\left(\frac{\Delta V_2}{\Delta V_1}\right)}{(P_{s1} - P_{s2})}, \text{ où}$$

$\Delta V_1$ - amplitude du signal du capteur de volume à contre-pression supérieure, déterminée pour chaque cycle cardiaque, $\Delta V_2$ - amplitude du signal du capteur de volume à contre-pression inférieure, déterminée pour chaque cycle cardiaque, $P_{s1}$ - valeur de la contre-pression supérieure appliquée par le capteur de volume pour chaque cycle cardiaque, $P_{s2}$ - valeur de la contre-pression inférieure appliquée par le capteur de volume pour chaque cycle cardiaque.

3. Procédé selon la revendication 1, **caractérisé en ce que**, pour chaque cycle cardiaque, la fonction de dépendance entre les courbes de pression et de volume est mise à jour et la pression pulsée est calculée selon la formule

$$PP = \frac{\Delta V_1}{k \cdot B \cdot e^{-k \cdot (P - P_{s1})}},$$

ou selon la formule

$$PP = \frac{\Delta V_2}{k \cdot B \cdot e^{-k \cdot (P - P_{s2})}},$$

où k est l'indice de compliance déterminé pour chaque cycle cardiaque, B est un paramètre déterminé par un étalonnage individuel préalable, $\Delta V_1$ - l'amplitude du signal du capteur de volume à contre-pression supérieure déterminée pour chaque cycle cardiaque, $\Delta V_2$ - l'amplitude du signal du capteur de volume à contre-pression inférieure déterminée pour chaque cycle cardiaque, $P_{s1}$ - la valeur de la contre-pression supérieure appliquée par le capteur de volume pour chaque cycle cardiaque, et $P_{s2}$ - la valeur de la contre-pression inférieure appliquée par le capteur de volume pour chaque cycle cardiaque.

4. Procédé selon les revendications 1 et 3, **caractérisé en ce que** la pression artérielle systolique pour chaque cycle cardiaque est calculée selon la formule

$$SBP = P + 0.5 \cdot PP,$$

et la pression artérielle diastolique selon la formule

$$DBP = P - 0.5 \cdot PP$$

**5.** Procédé selon la revendication 1, **caractérisé en ce que**, lors de la détermination de la pression artérielle pour chaque cycle cardiaque, les pressions appliquées par les capteurs de volume sont inférieures à la pression artérielle moyenne.

**6.** Procédé selon la revendication 1, **caractérisé en ce que**, pour déterminer le paramètre d'étalonnage individuel B, la pression appliquée par les capteurs de volume sur l'artère est augmentée au-dessus de la pression artérielle moyenne, tout en maintenant constante la différence entre les pressions appliquées par les capteurs de volume, pendant l'augmentation des contre-pressions, l'amplitude $\Delta V_{21}$ du signal différentiel entre les courbes de volume, l'indice de compliance k et les séries temporelles des contre-pressions $P_{s1}$, $P_{s2}$, sont calculés, à la fin de l'augmentation des contre-pressions, la valeur maximale $\Delta V_{21\_max}$ de la série temporelle des amplitudes $\Delta V_{21}$ du signal différentiel entre les courbes de volume ainsi que la valeur de l'indice de compliance $k_{max}$ correspondant à ce moment, et les pressions $P_{s1\_max}$, $P_{s2\_max}$ appliquées par les capteurs de volume sont déterminées selon la formule

$$B = \frac{\Delta V_{21\_max}}{(P_{s1\_max} - P_{s2\_max}) \cdot k_{max}},$$

ou la formule

$$B = \frac{\Delta V_{12\_max}}{(P_{s2\_max} - P_{s1\_max}) \cdot k_{max}}.$$

**7.** Procédé selon la revendication 1, **caractérisé en ce que**, pour déterminer le paramètre d'étalonnage individuel B, la pression artérielle systolique ($SBP_m$) et la pression artérielle diastolique sont mesurées à l'aide d'un dispositif externe de mesure de la pression artérielle et, simultanément à cette mesure, les séries temporelles des paramètres $\Delta V_{21}$ ou $\Delta V_{12}$, k, $P_{s1}$, $P_{s2}$, sont calculées, et après la mesure de la pression artérielle, les valeurs moyennes des séries temporelles des paramètres $\Delta V_{21\_m}$ ou $\Delta V_{12\_m}$, $k_m$, $P_{s1\_m}$, $P_{s2\_m}$ sont calculées selon la formule

$$B = \frac{\Delta V_{21\_m}}{(P_{s1\_m} - P_{s2\_m}) \cdot k_m \cdot e^{-k_m \cdot \left(DBP_m + 0.5 \cdot (SBP_m - DBP_m) - P_{s1\_m} + 0.5 \cdot (P_{s1\_m} - P_{s2\_m})\right)}},$$

ou la formule

$$B = \frac{\Delta V_{12\_m}}{(P_{s2\_m} - P_{s1\_m}) \cdot k_m \cdot e^{-k_m \cdot \left(DBP_m + 0.5 \cdot (SBP_m - DBP_m) - P_{s1\_m} + 0.5 \cdot (P_{s1\_m} - P_{s2\_m})\right)}}.$$

**8.** Dispositif de surveillance continue et non invasive de la pression artérielle, fondé sur la fonction de dépendance entre les courbes de pression et de volume pour l'estimation de la pression artérielle, comprenant deux capteurs optiques (1, 2) constitués chacun d'une source lumineuse (4, 5) et d'un photodétecteur (6, 7), des convertisseurs numérique-analogique (8) reliés aux sources lumineuses, des amplificateurs à transimpédance (9, 10) reliés électriquement aux photodétecteurs, des capteurs de force (12, 13) fixés aux capteurs optiques, des convertisseurs analogique-numérique (11) reliés électriquement aux capteurs de force et aux amplificateurs à transimpédance, un microcontrôleur (14) relié électriquement aux convertisseurs analogique-numérique et aux convertisseurs numérique-analogique, une mémoire (15) reliée électriquement au microcontrôleur, et un port de communication externe (16), **caractérisé en ce que** le boîtier du capteur (17) comprend des évidements pour un ou les deux optocoupleurs, afin de générer des différences de contre-pression exercées par les capteurs optiques.

**9.** Dispositif selon la revendication 8, dans lequel ledit capteur de force est muni d'un ressort, un premier ressort (21) étant monté entre le premier capteur optique (22) et le premier capteur de force (23), dont la raideur diffère de 0,1 à 2 fois par rapport à celle du second ressort monté entre le second capteur optique (25) et le second capteur de force (26), afin de créer des différences de contre-pression exercées par les capteurs optiques (1, 2).

**10.** Dispositif selon la revendication 8 ou 9, **caractérisé en ce que** le signal différentiel $V_{12}$ ou $V_{21}$ entre les courbes de volume et l'amplitude $\Delta V_{12}$ ou $\Delta V_{21}$ sont calculés dans le microcontrôleur pour la détermination de la pression artérielle.

**11.** Dispositif selon la revendication 10, **caractérisé en ce que** l'indice de compliance k de la fonction entre les courbes de pression et de volume est calculé dans le microcontrôleur pour chaque cycle cardiaque.

**12.** Dispositif selon la revendication 8 ou 9, **caractérisé en ce que** le dispositif passe automatiquement en mode d'étalonnage lorsqu'une augmentation des pressions mesurées par les capteurs de force est détectée ou lorsqu'il est commuté en mode d'étalonnage via le port de communication externe, et **en ce que** l'amplitude du signal différentiel $\Delta V_{12}$ ou $\Delta V_{21}$, l'indice de compliance k et les contre-pressions $P_{s1}$ et $P_{s2}$ sont enregistrés dans la mémoire reliée au contrôleur pour chaque cycle cardiaque et que leurs valeurs sont simultanément transmises via le port de communication externe.

**13.** Dispositif selon la revendication 12, **caractérisé en ce que** le dispositif détecte une diminution des pressions, proche du niveau initial, faisant suite à une augmentation des pressions mesurées par les capteurs de force, ce qui entraîne l'arrêt de l'enregistrement des paramètres, ou bien l'enregistrement est interrompu via le port de communication externe, à partir de la série temporelle des amplitudes $\Delta V_{12}$, l'amplitude maximale $\Delta V_{12\_max}$, la valeur correspondante de l'indice de compliance $k_{max}$ ainsi que les valeurs des pressions $P_{s1\_max}$ et $P_{s2\_max}$ appliquées par les capteurs de volume sont déterminées, et le paramètre d'étalonnage est calculé.

**14.** Dispositif selon la revendication 8 ou 9, **caractérisé en ce que** le dispositif est commuté en mode d'étalonnage via le port de communication externe, durant lequel les paramètres $\Delta V_{21}$ ou $\Delta V_{12}$, k, $P_{s1}$, $P_{s2}$ pour chaque cycle cardiaque sont enregistrés dans la mémoire reliée au microcontrôleur, et **en ce que** le dispositif est désactivé du mode d'étalonnage via ledit port de communication externe, et les valeurs de pression artérielle systolique ($SBP_m$) et diastolique ($DBP_m$), mesurées à l'aide d'un dispositif externe de mesure de la pression artérielle, sont introduites par ledit port, et sur la base des séries temporelles des paramètres enregistrés dans la mémoire, le microcontrôleur calcule, après la fin de la mesure de la pression artérielle, les valeurs moyennes $\Delta V_{21\_m} \Delta V_{12\_m}$, $k_m$, $P_{s1\_m}$. $P_{s2\_m}$, et calcule ensuite le paramètre d'étalonnage B.

**15.** Dispositif selon la revendication 14, **caractérisé en ce que** la pression artérielle P, la pression pulsée PP, la pression artérielle systolique SBP et la pression artérielle diastolique DBP sont calculées pour chaque cycle cardiaque dans le microcontrôleur du dispositif, et que ces valeurs sont respectivement transmises via le port de communication.

FIG 1

FIG 2

FIG 3

FIG 4

FIG 5

FIG 6

FIG 7

FIG 8

```
                        ┌──────────────────┐
                        │      Start       │
                        └────────┬─────────┘
                                 ▼
              ┌──────────────────────────────────┐
     ┌───────▶│ Detection of optical and applied │
     │        │         pressure signals         │
     │        └────────────────┬─────────────────┘
     │                         ▼
     │        ┌──────────────────────────────────┐
     │        │     Detection of cardiac cycle    │
     │        └────────────────┬─────────────────┘
     │                         ▼
     │        ┌──────────────────────────────────┐
     │        │ Calculation of parameters ΔV₂₁,  │
     │        │ ΔP₁₁₂, and compliance index k for │
     │        │           cardiac cycle           │
     │        └────────────────┬─────────────────┘
```

FIG 9

FIG 10

FIG 11

FIG 12

FIG 13

FIG 14

FIG 15

FIG 16

**Blood pressure (P): BIAS=0.011mmHg SD=4.2mmHg**

FIG 17

**Pulse pressure (PP): BIAS=0.003mmHg SD=4.7mmHg**

FIG 18

FIG 19

FIG 20

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5296310 A, Jones
- US 4846189 A, Sun Shuxing
- US 5423322 A, Clark
- WO 2017152098 A

**Non-patent literature cited in the description**

- **BAKER, P.D.** ; **WESTENSKOW, D.R** ; **KÜCK, K.** Theoretical analysis of non-invasive oscillometric maximum amplitude algorithm for estimating mean blood pressure. *Med. Biol. Eng. Comput.*, 1997, vol. 35, 271-278